# EUROPEAN PATENT SPECIFICATION

(11) **EP 1 399 074 B1**
(45) Date of publication and mention of the grant of the patent: **09.05.2007**
(21) Application number: 01949865.8
(22) Date of filing: 12.06.2001
(51) Int. Cl.: A61B 17/28, A61B 17/30

(54) **AUXILIARY FORCEPS FOR HAND-ASSISTED LAPAROSCOPIC SURGERY (HALS)**
HILFSZANGE FÜR MANUELL UNTERSTÜTZTE LAPARASKOPISCHE CHIRURGIE
PINCE AUXILIAIRE UTILISEE DANS LA CHIRURGIE LAPAROSCOPIQUE A LA MAIN (HALS)

(43) Date of publication of application: 24.03.2004
(73) Proprietor: SCUOLA SUPERIORE DI STUDI UNIVERSITARI E DI PERFEZIONAMENTO S. ANNA, 56127 Pisa (IT)
(72) Inventor: PIETRABISSA, Andrea, I-56122 Pisa (IT); STEFANINI, Cesare, I- 56023 Cascina (IT); MENCIASSI, Arianna, I-56025 Pontedera (IT); DARIO, Paolo, I-57100 Livorno (IT)
(74) Representative: Bardini, Marco Luigi
(86) International application number: PCT/IT2001/000298
(87) International publication number: WO 2002/100281

(56) References cited:
- WO-A-98/00069
- DE-A- 3 722 311
- US-A- 2 478 595
- US-A- 6 149 642
- US-A- 6 159 200
- US-B1- 6 174 321

## Description

### Field of the invention

The present invention relates to the field of the surgical instruments, particularly for the minimally invasive, video-assisted surgery (laparoscopic and toracoscopic surgery), and more precisely it relates to an auxiliary forceps for use in the surgical interventions carried out according to the hand-assisted laparoscopic surgery (HALS) technique.

### Background art

The technique of the hand-assisted laparoscopic surgery (HALS) uses the approach typical of the mini-invasive laparoscopic surgery combined with the classical approach of the open field surgery. As a matter of fact, according to the laparoscopic technique the access to the surgical field is carried out through applications of trocars (generally three accesses are sufficient) for the introduction of the instruments used during the intervention and of the laparoscope, of the optical fiber type or equipped with a CCD camera, by means of which the surgeon can visually control the involved inner part of the abdomen. The diameter of the incisions is comprised between 5 and 25 mm. A working space, called pneumaperitoneum, is created in the abdomen by insufflating an inert gas to enable the surgeon to operate without significant hindrances due to the lack of suitable space. In addition to the above described incisions, in the hand-assisted laparoscopic surgery a further incision 5-7 cm long is made near them, through which the surgeon introduces his/her, generally non-dominant, hand in the abdomen to perform support operations (tissue displacement, retraction of the organs to be operated, palpation, interaction with other instruments, etc.).

In order to prevent the pneumaperitoneum to be lost through this incision, several types of sealing devices are available, intended to be applied to the incision to allow the passage of the surgeon's hand, while preventing the gas from escaping therethrough. The presently available sealing devices are of several types, in particular of the type comprising an adhesive flange to be fixed to the abdominal wall or of the inflatable type. A sealing device of the inflatable type especially easy and comfortable in use is disclosed in WO00/32117. It consists of a substantially tubular inflatable sleeve with a twisted inner cross section, to provide sealing against the surgeon's arm once her/his hand has been inserted through it, and a pair of sealing rings to provide a sealing from the innerside and the outerside of the abdominal wall in correspondence to the incision.

Hand-assisted laparascopic surgery has been successfully applied to a wide range of surgical procedures for example gastric resection, gastric by-pass, transhiatal esophgectomy, pancreatic and hepatic surgery, nephrectomy, colorectal surgery, aortic aneurysm repairs, etc. The main advantages of this technique with respect to the conventional laparoscopic surgery, consist, as well known, in that the surgeon regains direct tactile sensation, not allowed when remotely controlled instruments are used, and hand-eye coordination which is lost when procedures are followed through a monitor. Furthermore the presence of the surgeon's hand in the surgical field allows for an easier and atraumatic displacement of the organs, an immediate control of dangerous situations and the possibility of performing blunt dissections. In addition, the possibility of using an assisting hand makes easier the surgeon task reducing the high level of training and experience usually required by the laparoscopic surgery technique.

However, in the course of hand-assisted laparoscopic surgery procedures situations often occur wherein the surgeon's hand ability is no longer adequate to perform fine operations such as fine dissections, vascular peduncula isolation, limphadenectomy, etc. In the conventional open field surgery, when a tissue to be grasped has a size of few millimeters, such that the surgeon's finger tips would be ineffective, the use of a forceps, generally operated by the non-dominant hand, does not constitute a problem. In contrast in a minimally invasive surgery procedure, such as the hand-assisted laparoscopic surgery, the use of the conventional surgical forceps is not possible both in view of their shape, incompatible with the space available in the surgical field, and taking into account of the way this type of intervention is carried out, according to which it is advisable to reduce to a minimum the number of times the non-dominant hand is extracted and re-inserted in the surgical field.

WO-A-980069 which corresponds to the preamble of claim 1, discloses a tiny surgical instrument directly mounted on a surgeon's fingertips in a way that the surgeon can insert his/her hand into the patient to perform surgical procedures and also to use his/her fungers to manipulate tissues. The instrument is provided with a tool and a mount for securing the tool to a fingertip and a retracting/deploying mechanism operably connected to the mount to permit the tool to be moved into a retracted position relative to the mount to expose the fingertip and into a deployed position relative to the mount for use of the tool. In this way, the tool is always available for use in surgical field and can be moved into a retracted position to allow the surgeon to manipulate tissues with the non-dominant hand.

However, with this prior art instrument the surgeon's hand is not completely free when he/she does not need to use it, the tool being directly mounted on a fingertip. Even if the tool can be placed in a retracted, non-use position, it may be of hindrance to the movements of the fingers and the hand of the surgeon especially in such a very narrow space as in the case of a laparoscopic surgery.

To keep the surgeon's hands always free the same applicants of WO-A-980069 have suggested in US-A-6159200 to use an instrument exchange cannula through which a suitable miniature instrument can be deployed in, or retracted from, the surgical field. In this case the tool is moved into the cannula through a tether to which it is connected at one of its ends. Even if the movements of the surgeon's hand are not hindered by tools attached thereto, the advantage of having the proper tool always ready and available at the surgical field is lost and a surgeon's assistant must enter the tool into the body cavity when needed. Furthermore, in this case an additional access port for the insertion of the instrument exchange cannula must be created on the abdominal wall.

### Summary of the invention

The main object of the present invention is to provide a structurally simple, reliable and low dimension forceps for surgical use, which can be used by the surgeon, when necessary, in video-assisted, minimally invasive surgical procedures, in particular according to the hand-assisted laparoscopic technique.

A particular object of the present invention is to provide a forceps of the above mentioned type, whose structure, shape and dimensions is such that it can be kept at the surgeon's disposal in the abdominal cavity during the intervention in such a position that it can be quickly and easily grasped with the fingers by the surgeon, once it is necessary or, it can be parked after the use without any risk of accidentally damaging the surrounding organs and tissues.

Another object of the present invention is to provide a forceps of the above mentioned type which is able to be kept integral to the surgeon's hand and, when not in use, during the intervention can be parked in the abdominal cavity with no risk that its jaws can constitute a danger of possible lesions to the surrounding structures.

A further object of the present invention is to provide a forceps of the above mentioned type which is so configured that it can be operated with a minimum number of fingers (two).

Another object of the present invention is to provide a method for performing surgical procedures with a hand-assisted laparoscopic technique, which thanks to the use of the auxiliary forceps of the above mentioned type, makes easier the surgeon's task and allows the accomplishments of operations which would be impossible to perform by manual way only.

The above objects are reached with the auxiliary forceps for video-assisted, minimally invasive surgery, such as the hand-assisted laparoscopic surgery, according to the invention as claimed in claim 1, which is formed by two actuating plates, elastically hinged to each other and having a shape and dimensions such that they can be operated by keeping them between the tips of two fingers, and by two jaws each extending from a respective of said actuating plates. The forceps further comprises means for the temporarily connection to the surgeon's hand for maintaining the forceps at an adjustable distance from the hand not greater than a prefixed value and means for housing the jaws in a mutually side-by-side condition, within said housing means the jaws being engaged when, during the intervention, the forceps is not used.

According to the invention, the forceps is fixed to the surgeon's hand by means of a rope connected to the plates and preferably having an anchoring device such as a loop made with the same rope, an elastic ring or a clamp having adjustable width and distance from the plates.

In particular, the housing for the jaws can be formed by a removable protective cap connected to the plates by means of an adjustable length rope, or by a seat perimetrically formed at the edge of the plates, in which case the jaws are of the retractable type as a result of an angular displacement from an operating position to a rest position within said seat and have a curved shape.

### Brief description of the drawings

Those and other features and advantages of the auxiliary forceps for video-assisted, minimally invasive surgery such as the hand-assisted laparoscopic surgery will become apparent from the following description of some exemplifying, non-limiting embodiments thereof, made with reference to annexed drawings, wherein:
- figure 1 is an assembly view of a first embodiment of the auxiliary forceps according to the invention;
- figure 2 is an enlarged perspective view of the forceps of figure 1;
- figure 3 is a diametrical section of the forceps of figure 2 according to the axis of the jaws;
- figures 4 and 5 show in a perspective view a second embodiment of the forceps according to the invention, in a closed and, respectively, open condition;
- figure 6 is a diametrical section of the forceps according to line VI-VI of figure 4;
- figure 7 is a partially sectioned, plan view of the forceps of figure 5.

### Description of the preferred embodiment

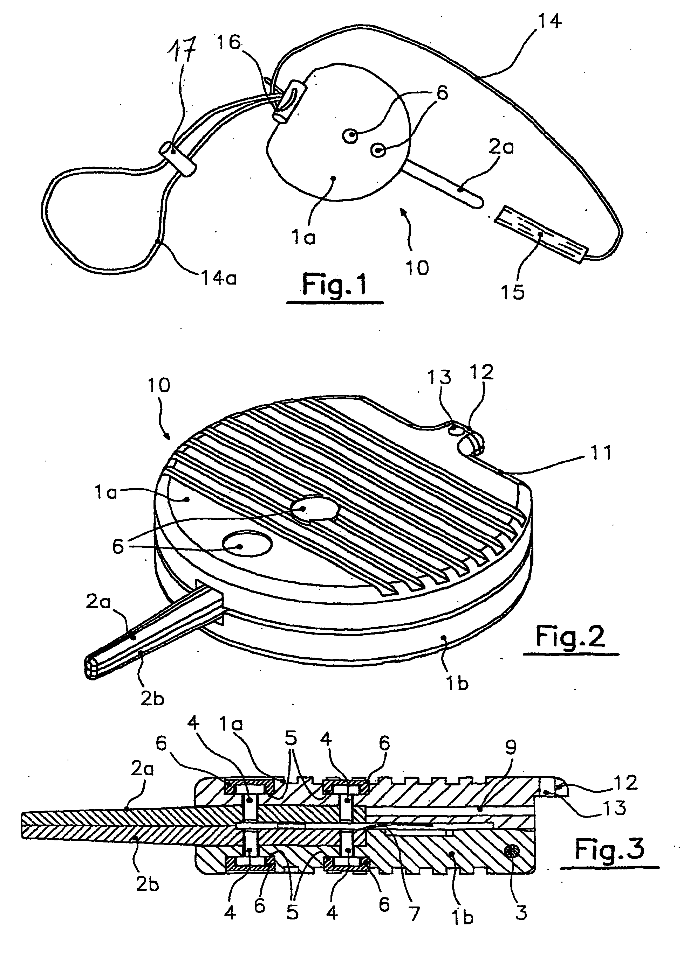

With reference to figures 1, 2 and 3, 10 generally indicates a forceps formed by a pair of substantially circular actuating plates 1a, 1b connected to each other by means of a pin 3 extending at a chord thereof. Actuating plates 1a, 1b are of a very low size, sufficient to be grasped and actuated by holding them between the tips of two fingers, which corresponds to a few centimer diameter (for example, 3 cm). Respective jaws 2a, 2b are fixedly connected to plates 1a, 1b in any way, for example, as shown in the figures, by means of screws 4, and extend from the part diametrically opposed to pin 3 and perpendicularly to it. The heads of screws 4 are housed within respective seats 5 formed on plates 1a, 1b and concealed by covering caps 6, for example snap-fitted to them. A spring 7, for example of the flat type, is interposed between plates 1a and 1b for keeping jaws 2a, 2b spaced apart. A diametrical channel 9, extending from the perimetrical edge of plate 1a to the inner end of jaw 2a is formed in plate 1a to connect jaw 2a through an electric cable to an electric power source, for example to enable the forceps to be used to perform unipolar or bipolar electrocoagulation.

A bracket 12 with a through hole 13 extends from a facing 11 formed on the perimetrical edge of plate 1a (plate 1b has a corresponding facing), from the part diametrically opposed to jaw 2a. A rope 14 is tied to hole 13 and a cap 15 is fixed to rope 14 for acting as a sheath for the pair of jaws 2a, b of the forceps, when it is not in use. Rope 14 forms a loop 14a of adjustable width by sliding a rope-clamping slider 17. A second slider 16, engaged with rope 14 immediately dowstream of bracket 12, assists in adjusting the length of the rope at the end of which cap 15 is fixed. As an alternative to loop 14a with a fully equivalent function, rope 14 can be provided with an elastic ring or a fixing clamp.

In the use, forceps is fixed by means of loop 14a to a phalanx or the wrist of the surgeon's non-dominant hand, before inserting the hand in the abdomen, while jaws 2a, 2b are placed in cap 15, whereby any risk of lesions accidentally caused to the surrounding tissues is avoided and, at the same time, jaws 2a, 2b are kept close to each other thus overcoming the bias of spring 7 and keeping forceps 10 closed. When the forceps must be used, the surgeon takes cap 15 off and operates the forceps by holding actuating plates between two finger tips. In order to make the grasp between the fingers easier, the surface of plates 1a, b can be variously processed, for example by grooving, as in the present embodiment, or ribbing, curling and the like.

When during a surgery procedure, forceps is not used, it anyway remains anchored to the surgeon's hand through rope 14 at a distance not greater than the length of the rope portion comprised between rope clamps 17 and 16 and then at a distance fixable according to the needs.

In a possible variation, not shown, protective cap 15 can be replaced by a substantially C-shaped sheath pivotally connected to one of the plates. By rotation of the sheath jaws can be engaged within C-shaped sheath or disengaged from it, thus achieving the same function of cap 15.

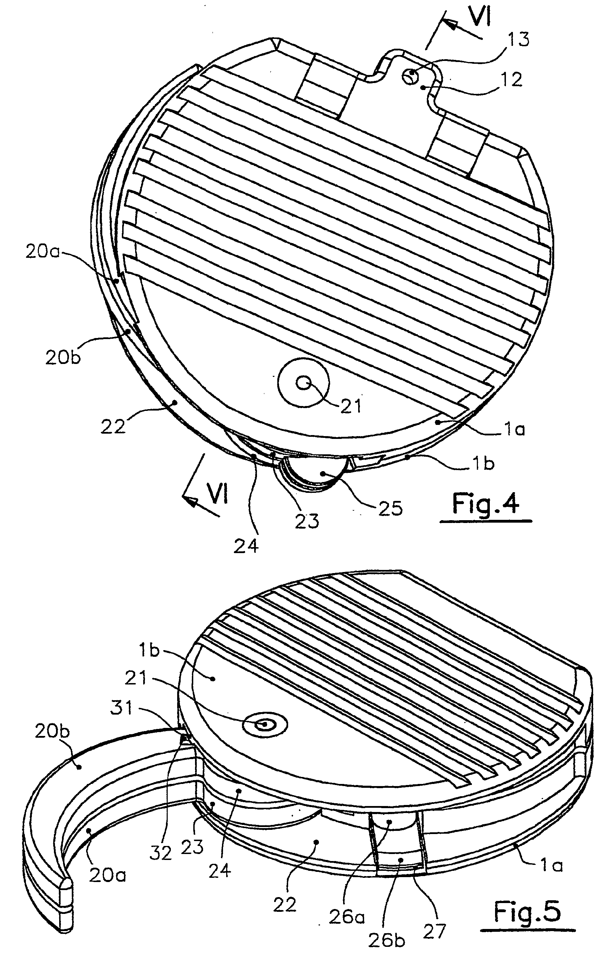

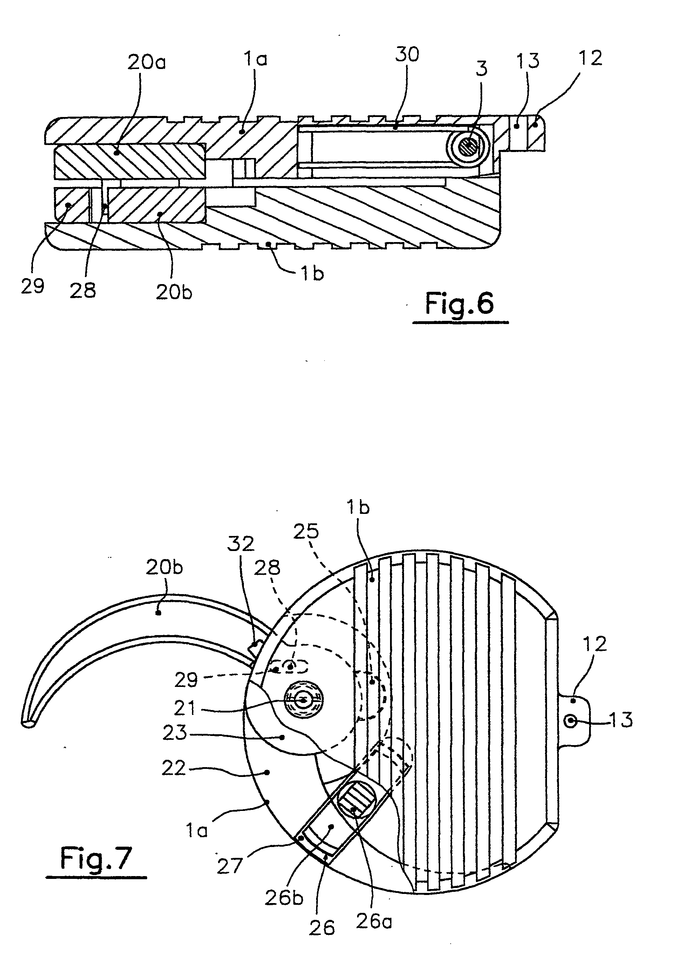

A second embodiment of the invention is shown in figures 4, 5, 6 and 7, wherein the same components of the forceps as those of forceps of figures 1-3 are referred with the same numerals. The feature of the forceps according to this embodiment of the invention consists in that its jaws, indicated at 20a and 20b, are retractibly housable within a housing seat 22 formed on perimetrical edge of actuating plates 1a and 1b. As a consequence, jaws 20a and 20b have a curved shape extending from respective enlarged portion, or root, 23 and 24, at which they are hinged to actuating plates 1a and 1b by means of respective pins 21. Actuating plates 1a and 1b are hinged to each other through a pin 3, as in the previous embodiment, and elastic means 30, for example as shown in figure 6, are provided therebetween to keep the forceps open.

As shown in figure 4, jaws 20a, 20b are formed at respective roots 23 and 24 with a side protrusion 25 which allows a tangential force to be exerted on roots 23 and 24 to release jaws 20a, 20b and let them reach the working position shown in figure 5.

In order to keep the forceps closed when jaws 20a, 20b are in their rest position shown in figure 4, a fastening device is provided between the actuating plates and constituted, in the present embodiment of the invention, by a substantially T-shaped bracket 26 fixed to one of the plates, for example plate 1b, by its leg 26a and extending with its transverse portion 26b within seat 22 in a groove 27 correspondingly formed in the other plate, for example plate 1a. In this way, when jaws 20a, 20b are placed in housing seat 22, the jaw integral to the plate within which groove 27 is formed, abuts on the transverse part 26b of bracket 26, whereby it cannot escape from groove 27 and, as a result, the plates are secured to each other.

In order to ensure a perfect mutual parallelism of jaws 20a and 20b in any of their positions, a pin 28 (figures 6 and 7) extends from a plate towards the other one and engages within a slot 29 formed in the root of the latter plate. Slot 29 is so oriented as to be perpendicular to the forceps hing when jaws are in the operating position. In this way, pin 28 can freely tilt within slot 29 when the jaws are made to space apart and keep them secured to each other when they are closed.

As a consequence of the force exterted on jaws 20a, 20b, when they are in the retracted position, by elastic means 30 through transverse portion 26b of bracket 26, the forceps is normally kept closed and it is necessary to slightly urge against protrusion 25 to open it. In order to keep the jaws in their operating position, an end-stroke locking device is provided, which is constituted, in the present embodiment, by a tooth 31 (figures 5 and 7), protruding from one of the plates towards the other one and snap engaging within a cavity 32 correspondingly formed on the jaws integral to the same plate. Cavity 32 and/or tooth 31 can be formed with inclined walls or sides to make easier the snap engagement/disengagement taking also advantage of the elasticity of the material. Other types of end-stroke locking device, fully equivalent from the functional point of view, can be provided in a obvious way.

With the forceps according to the present embodiment of the invention there is no need for its jaws to be capped, because, when not in use, they are retractably housed in seat 22 perimetrically formed on edges of plates 1a, 1b. To secure the forceps to the surgeon's hand, the same solution as shown for forceps of figures 1-3 can be adopted consisting in the use of a rope 14 tied to bracket 12 through hole 13 and forming a loop 14a of a diameter which is adjustable by a means of a rope clamp slider 15.

Another way to secure the auxiliary forceps according to the invention to the surgeon's hand consists in providing a rope-anchoring adhesive member to be applied to the glove worn by the surgeon in such a position not to hinder the hand movements, for example on the back of the hand, or again at the base of a finger phalanx like a ring or hemi-ring with adhesive plate.

The facing surfaces of the jaws of the forceps according to the invention are shaped in a substantially conventional way according to the type of function they are designed to perform; for example they can be formed with a toothed surface to better grasp the tissues and/or with a suitable seat to hold a needle thereby performing the function of a needle holder, or with other shapes resulting in an obvious way from the specific function.

The use of the auxiliary forceps according to the present invention results in a number of advantages in the hand-assisted laparoscopic surgery. As a matter of fact, the forceps of the invention allows a number of operations to be performed which cannot carried out with the only help of the surgeon hand (vascular peduncula isolation, lynphoglandula ablation, grasping of small portions of tissues). The small size of the forceps (the operating plates 1a, 1b are of a diameter of few centimeters) give rise to a minimum hindrance, still they allow an easy and firm handling of the forceps when the plates are grasped with two fingers. In fact, they have a wide actuating surface for the fingers which makes unnecessary a third fulcrum (for example the hollow at the thumb root), as requested with the conventional, elongated structure forceps.

The substantially circular shape, with streamlined and/or rounded off edges, makes also extremely safe the use thereof, which is practically riskless as regards possible accidental lesions to the tissues. Furthermore, it has to be pointed out that, in the case of the embodiment of the forceps in which the jaws are curved, the curved shape of the jaws offer the advantage of a minimum hindrance when the instrument is its rest condition, and allows for the use of the forceps both with a grasping and a dissecting effect on the tissues, with the possibility of surrounding vascular structures according to techniques well-known in the field.

The forceps according to the invention is designed to be used as a disposable instrument. However, it can be made of materials able to be subjected to sterilization according to known techniques and therefore to be re-used. Preferably, the operating plates will be made of a dark color or a transparent color, to avoid unwanted reflections which could disturb the sight of the surgical field through a monitor. The transparency of the plates also allows the sight of the otherwise concealed portion of the surgical field to be maintained through the plates.

Variations and/or modifications can be brought to the auxiliary forceps for hand-assisted laparoscopic surgery according to the present invention, without departing from the scope of the invention as defined in the appended claims.

## Claims

1. Auxiliary forceps for video-assisted, minimally-invasive surgery, such as the hand-assisted laparoscopic surgery, intended to be operated by the surgeon's non-dominant hand inserted in an incision for entering a patient body natural cavity, comprising two actuating parts (1a, 1b) elastically hinged to each other and having a shape and size suitable for being operated by holding them with the tips of two fingers, and two jaws (2a, b; 20a, b) each extending from a respective of said actuating plates, **characterized in that** said two actuating parts are two actuating plates (1a, 1b) and **in that** rope means (14) extending from said plates are provided for temporarily connecting the forceps to the surgeon's hand while maintaining the forceps at an adjustable distance from the hand not greater than a prefixed value, whereby the forceps can be easily retrieved when its use is required, and means (15, 22) for housing said jaws close to each other, connected to, or formed in, said plates, within which said jaws are engaged when, during operation, said forceps is not used.

2. Forceps according to claim 1, wherein said rope means comprise a rope (14) which can be connected to said actuating plates and provided with a connection device (14a), adjustable as to its width and distance from said plates, for being applied to a finger or the wrist of the surgeon's non-dominant hand.

3. Forceps according to claim 2, wherein said connection device is a loop (14a) made with the same rope (14).

4. Forceps according to claim 2-, wherein said connection device is an adhesive member, applied to the surgeon's glove in such position not to hinder the movements of the hand to which said rope is fixed.

5. Forceps according to anyone of the previous claims, wherein said jaws (2a, b) are firmly connected to the respective actuating plates (1a, b) and said housing means for the jaws comprise a protective cap (15) to be removably applied to them and connected to said plates through a rope (14) of adjustable length.

6. Forceps according to anyone of the claims 1 to 4, wherein said jaws (2a, b) are pivotally connected to the respective actuating plates (1a, b) and said housing means comprise a seat (22) formed along the perimetrical edge of said plates, said jaws having a correspondingly curved shape to be placed within said seat in a rest position following an angular displacement from an operating position, wherein said jaws extend in a substantially radial direction from said plates, said jaws being formed with a side protrusion (25) through which a force for causing said angular displacement from the rest position to the operating position can be exerted.

7. Forceps according to claim 6, wherein fastening means (26) are provided for keeping said actuating plates close to each other when said jaws are in their rest position.

8. Forceps according to claim 7, wherein said fastening means comprise a bracket (26) integral to one of said plates and extending toward the other plate and having an arm (26b) arranged in a groove (27) formed in the other plate in said housing seat (22) for the jaws, whereby, when said jaws are engaged in said housing seat (22), the jaw integral to the plate, in which the groove (27) for said arm is formed, abuts against the arm integral to the other plate, thus anchoring said plates to each other.

9. Forceps according to anyone of the claims 6, 7 or 8, wherein a slot (29) is formed on one of said jaws in such a way to be arranged in a position substantially orthogonal to the hinge axis of said actuating plates, when said jaws are in the operating position, a pin (28) extending from the other jaw for engaging within said slot to prevent any relative sliding of the jaws when they are angularly displaced between the rest position and the operating position, while allowing their spreading apart during the use.

10. Forceps according to anyone of the claims 6 to 9, wherein reversible locking means (31, 32) of the jaws in the operating position and in the rest position are provided.

11. Forceps according to claim 10, wherein said means for reversibly locking said jaws in the operating position comprise a cavity (32) formed in at least one of said jaws and a tooth (31) correspondingly extending from the respective actuating plate, for reversibly snap engaging within said cavity when said jaws reach their operating position.

12. Forceps according to claim 10, wherein elastic means are provided between said actuating plates (1a, b) for keeping them spread apart and urging the arm (26b) of said bracket (26) against said jaws (20a, b) to reversibly fasten them in the rest position.

13. Forceps according to claim 1, wherein said housing means comprise a sheath connected to one of said plates and turnable to cover said jaws following an angular displacement when said forceps is not in use.

14. Forceps according to anyone of the previous claims, wherein said actuating plates are made of transparent or dark colored material.

15. Forceps according to anyone of the previous claims, wherein said actuating plates have a substantially circular shape and rounded edges.

## Patentansprüche

1. Hilfszange zur videounterstützten minimalinvasiven Chirurgie, wie die manuell unterstützte laparoskopische Chirurgie, vorgesehen, um mit der nicht dominanten Hand des Chirurgen bedient zu werden, eingeführt in einen Schnitt zum Eindringen in einen natürlichen Hohlraum eines Patientenkörpers, enthaltend zwei Betätigungsteile (1a, 1b), die elastisch aneinander angelenkt sind und eine Form und Größe haben, die geeignet ist, um, indem sie mit den Spitzen von zwei Fingern gehalten werden, bedient zu werden, und zwei Backen (2a, b; 20a, b), von denen sich jede von einem entsprechenden der Betätigungsteile erstreckt, **dadurch gekennzeichnet, dass** die zwei Betätigungsteile zwei Betätigungsplatten (1a, 1b) sind, und dass Schnureinrichtungen (14), die sich von den Platten aus erstrecken, zum temporären Verbinden der Zange mit der Hand eines Chirurgen während die Zange in einem einstellbaren Abstand von der Hand nicht größer als ein vorbestimmter Wert beibehalten wird, wodurch die Zange leicht zurück erhalten werden kann, wenn ihre Anwendung erforderlich ist, und, um die Backen nahe beieinander unterzubringen, Einrichtungen (15, 22) vorgesehen sind, die verbunden sind mit oder ausgebildet sind in den Platten, worin die Backen in Eingriff sind, wenn die Zange während des Betriebes nicht verwendet wird.

2. Zange nach Anspruch 1, wobei die Schnureinrichtungen eine Schnur (14) enthalten, die mit den Betätigungsplatten verbunden werden kann und mit einer Verbindungsvorrichtung (14a) versehen ist, die hinsichtlich ihrer Weite und ihres Abstandes von den Platten einstellbar ist, um an einen Finger oder das Handgelenk der nicht dominanten Hand des Chirurgen angelegt zu werden.

3. Zange nach Anspruch 2, wobei die Verbindungsvorrichtung eine Schlaufe (14a) ist, die aus derselben Schnur (14) besteht.

4. Zange nach Anspruch 2, wobei die Verbindungsvorrichtung ein Adhäsivglied ist, das an dem Handschuh des Chirurgen in einer solchen Position angelegt wird, um die Bewegungen der Hand nicht zu behindern, an welcher die Schnur befestigt ist.

5. Zange nach einem der vorhergehenden Ansprüche, wobei die Backen (2a, b) fest mit den jeweiligen Betätigungsplatten (1a, b) verbunden sind und die Unterbringungseinrichtungen für die Backen eine Schutzkappe (15) enthalten, um entfernbar an ihnen angelegt zu werden und mit den Platten durch eine Schnur (14) von einstellbarer Länge verbunden zu werden.

6. Zange nach einem der Ansprüche 1 bis 4, wobei die Backen (2a, b) drehbar mit den jeweiligen Betätigungsplatten (1a, b) verbunden sind und die Unterbringungseinrichtungen einen Sitz (22) enthalten, der längs des Umfangsrandes der Platten ausgebildet ist, welche Backen eine entsprechend gekrümmte Form haben, um innerhalb des Sitzes in einer Ruheposition nachfolgend einer Winkelversetzung aus einer Betriebsposition angeordnet zu werden, wobei die Backen sich in einer im wesentlichen radialen Richtung von den Platten erstrecken, welche Backen mit einem Seitenvorsprung (25) ausgebildet sind, durch welchen eine Kraft zum Verursachen der Winkelversetzung aus der Ruheposition in die Betriebsposition ausgeübt werden kann.

7. Zange nach Anspruch 6, wobei Befestigungseinrichtungen (26) vorgesehen sind, um die Betätigungsplatten nahe aneinander zu halten, wenn die Backen in ihrer Ruheposition sind.

8. Zange nach Anspruch 7, wobei die Befestigungseinrichtungen eine Klammer (26) enthalten, die integral mit einer der Platten ist und sich zu der anderen Platte hin erstreckt und einen Arm (26b) hat, der in einer Nut (27) angeordnet ist, die in der anderen Platte in dem Unterbringungssitz (22) für die Backen ausgebildet ist, wodurch, wenn die Backen in dem Unterbringungssitz (22) in Eingriff sind, die Backe, die integral mit der Platte ist, in welcher die Nut (27) für den Arm ausgebildet ist, gegen den Arm anliegt, der integral mit der anderen Platte ist, wodurch die Platten aneinander verankert werden.

9. Zange nach einem der Ansprüche 6, 7 oder 8, wobei ein Schlitz (29) in einer der Backen in einer solchen Weise ausgebildet ist, um in einer Position im wesentlichen orthogonal zu der Gelenkachse der Betätigungsplatten angeordnet zu sein, wenn die Backen in der Betriebsposition sind, wobei sich ein Stift (28) von der anderen Backe aus erstreckt, um innerhalb des Schlitzes in Eingriff zu sein, um jegliches relatives Verschieben der Backen zu verhindern, wenn sie winkelmäßig zwischen der Ruheposition und der Betriebsposition versetzt werden, während ihr Auseinanderspreizen während der Anwendung gestattet ist.

10. Zange nach einem der Ansprüche 6 bis 9, wobei reversible Verriegelungseinrichtungen (31, 32) der Platten in der Betriebsposition und in der Ruheposition vorgesehen sind.

11. Zange nach Anspruch 10, wobei die Einrichtungen zum reversiblen Verriegeln der Backen in der Betriebsposition einen Hohlraum (32), der in wenigstens einer der Backen ausgebildet ist, und einen Zahn (32) enthalten, der sich entsprechend von der entsprechenden Betätigungsplatte aus erstreckt, um reversibel innerhalb des Hohlraums in Schnappeingriff zu sein, wenn die Backen ihre Betriebsposition erreichen.

12. Zange nach Anspruch 10, wobei Elastikeinrichtungen zwischen den Betätigungsplatten (1a, b) vorgesehen sind, um sie auseinander gespreizt zu halten und den Arm (26b) der Klammer (26) gegen die Backen (20a, b) zu drängen, um sie reversibel in der Ruheposition festzumachen.

13. Zange nach Anspruch 1, wobei die Unterbringungseinrichtungen eine Hülle enthalten, die mit einer der Platten verbunden ist und drehbar ist, um die Backen auf eine Winkelversetzung folgend abzudecken, wenn die Zange nicht in Gebrauch ist.

14. Zange nach einem der vorhergehenden Ansprüche, wobei die Betätigungsplatten aus transparentem oder dunkel gefärbtem Material hergestellt sind.

15. Zange nach einem der vorhergehenden Ansprüche, wobei die Betätigungsplatten eine im wesentlichen kreisartige Form und abgerundete Ränder haben.

## Revendications

1. Pince auxiliaire pour la chirurgie mini-invasive assistée par vidéo, telle que la chirurgie laparoscopique assistée à la main, prévue pour être actionnée par la main non-dominante du chirurgien insérée dans une incision pour entrer dans une cavité naturelle du corps d'un patient, comprenant deux parties d'actionnement (1a, 1b) montées en rotation de façon élastique l'une par rapport à l'autre et ayant une forme et des dimensions adaptées pour être commandées en les maintenant avec le bout de deux doigts, et deux mâchoires (2a, b ; 20a, b) s'étendant chacune à partir d'une partie d'actionnement respective, **caractérisé en ce que** les deux dites parties d'actionnement sont deux plaques d'actionnement (1a, 1b) et **en ce que** des moyens de lien (14) s'étendant à partir des dites plaques sont agencés pour relier temporairement la pince à la main du chirurgien tout en maintenant la pince à une distance de la main ajustable non supérieure à une valeur prédéterminée, grâce à quoi la pince peut être facilement retrouvée quand on veut l'utiliser, et **en ce que** des moyens (15, 22) pour loger lesdites mâchoires l'une contre l'autre, sont reliés aux dites plaques, ou réalisés dans lesdites plaques, dans lesquels lesdites mâchoires sont logées quand, pendant l'opération, ladite pince n'est pas utilisée.

2. Pince selon la revendication 1, dans laquelle lesdits moyens de lien comportent un lien (14) qui peut être relié aux dites plaques d'actionnement et qui est muni d'un dispositif de raccordement (14a), réglable quant à sa largeur et sa distance aux dites plaques, pour être relié à un doigt ou au poignet de la main non-dominante du chirurgien.

3. Pince selon la revendication 2, dans laquelle ledit dispositif de raccordement est une boucle (14a) réalisée avec le même lien (14).

4. Pince selon la revendication 2, dans laquelle ledit dispositif de raccordement est un élément adhésif, appliqué sur le gant du chirurgien dans une position qui ne gêne pas les mouvements de la main à laquelle ledit lien est fixé.

5. Pince selon l'une quelconque des revendications précédentes, dans laquelle lesdites mâchoires (2a, b) sont fermement connectées respectivement aux plaques d'actionnement (1a, b) et lesdits moyens de logement pour les mâchoires comportent un étui de protection (15) apte à être enfiché sur elles de façon amovible et relié aux dites plaques via un lien (14) de longueur ajustable.

6. Pince selon l'une quelconque des revendications 1 à 4, dans laquelle lesdites mâchoires (2a, b) sont montées pivotantes respectivement sur les plaques d'actionnement (1a, b) et lesdits moyens de logement comportent un siège (22) réalisé le long du bord périphérique des dites plaques, lesdites mâchoires ayant une forme incurvée correspondante pour être positionnées dans ledit siège dans une position de repos après un déplacement angulaire à partir d'une position opérationnelle, dans laquelle lesdites mâchoires se prolongent dans une direction sensiblement radiale à partir des dites plaques, lesdites mâchoires comportant une saillie latérale (25) sur laquelle une force peut être exercée pour entraîner leur déplacement angulaire de leur position de repos à leur position opérationnelle.

7. Pince selon la revendication 6, dans laquelle des dispositifs de blocage (26) sont prévus pour maintenir les plaques d'actionnement l'une contre l'autre quand lesdites mâchoires sont dans leur position de repos.

8. Pince selon la revendication 7, dans laquelle lesdits dispositifs de blocage comportent un crochet (26) faisant partie intégrante de l'une des dites plaques et s'étendant vers l'autre plaque et comprenant un bras (26b) agencé dans une gorge (27) réalisée dans l'autre plaque dans ledit siège de logement (22) pour les mâchoires, de façon que, lorsque lesdites mâchoires sont engagées dans ledit siège de logement (22), la mâchoire faisant partie intégrante de la plaque dans laquelle a été réalisée la gorge (27) pour ledit bras, vient buter contre le bras faisant partie intégrante de l'autre plaque, pour ainsi ancrer lesdits plaques entre elles.

9. Pince selon l'une quelconque des revendications 6, 7 et 8, dans laquelle une fente (29) est réalisée sur l'une des dites mâchoires de façon à être dans une position sensiblement orthogonale à l'axe de rotation des dites plaques d'actionnement, quand lesdites mâchoires sont dans la position opérationnelle, une goupille (28) s'étendant à partir de l'autre mâchoire pour s'engager dans ladite fente dans le but d'empêcher tout glissement relatif des mâchoires quand elles sont déplacées angulairement entre leur position de repos et leur position opérationnelle, tout en permettant leur écartement pendant leur utilisation.

10. Pince selon l'une quelconque des revendications 6 à 9, dans laquelle sont prévus des moyens de blocage réversible (31, 32) des mâchoires dans leur position opérationnelle et dans leur position de repos.

11. Pince selon la revendication 10, dans laquelle lesdits moyens de blocage réversible des dites mâchoires de leur position opérationnelle comportent une cavité (32) réalisée dans au moins l'une des dites mâchoires et un ergot (31) s'étendant radialement de façon complémentaire à partir de la plaque d'actionnement correspondante, pour s'engager vivement de façon réversible dans ladite cavité quand lesdites mâchoires atteignent leur position opérationnelle.

12. Pince selon la revendication 10, dans laquelle sont prévus des moyens élastiques entre lesdites plaques d'actionnement (1a, b) pour les maintenir écartées l'une de l'autre et pour fixer le bras (26b) du dit crochet (26) contre lesdites mâchoires (20a, b) pour les fixer de façon réversible dans leur position de repos.

13. Pince selon la revendication 1, dans laquelle lesdits moyens de logement comportent un capot relié à l'une des dites plaques et pouvant être pivoté pour recouvrir lesdites mâchoires après un déplacement angulaire quand ladite pince n'est pas utilisée.

14. Pince selon l'une quelconque des revendications précédentes, dans laquelle lesdites plaques d'actionnement sont réalisées en un matériau transparent ou de couleur foncée.

15. Pince selon l'une quelconque des revendications précédentes, dans laquelle lesdites plaques d'actionnement sont d'une forme sensiblement circulaire à bords arrondis.
